# EUROPÄISCHE PATENTSCHRIFT

(11) **EP 0 355 307 B1**
(45) Veröffentlichungstag und Bekanntmachung des Hinweises auf die Patenterteilung: **28.12.1994**
(21) Anmeldenummer: 89111285.6
(22) Anmeldetag: 21.06.1989
(51) Int. Cl.: C12P 7/62

(54) **Extraktionsmittel für Poly-D(-)-3-hydroxybuttersäure**
Extraction agent for poly-D(-)-3-hydroxybutyric acid
Moyens d'extraction de l'acide poly-D(-)-3-hydroxy butyrique

(30) Priorität: 07.07.1988 AT 1759/88
(43) Veröffentlichungstag der Anmeldung: 28.02.1990
(73) Patentinhaber: PCD-Polymere Gesellschaft m.b.H., A-2323 Schwechat-Mannswörth (AT)
(72) Erfinder: Traussnig, Heinz, Dipl. Ing., A-8130 Frohnleiten (AT); Kloimstein, Engelbert Ing., A-4070 Eferding (AT); Kroath, Hans, Dr., A-4040 Linz (AT); Estermann, Robert, A-4020 Linz (AT)
(74) Vertreter: Kunz, Ekkehard, Dr.

(56) Entgegenhaltungen:
- US-A- 4 140 741
- ANGEWANDTE CHEMIE, Band 74, Nr. 10, 1962, Weinheim (DE); H.G. SCHLEGEL et al., Seiten 342-347#
- CHEMICAL ABSTRACTS, Band 100, 1984, Columbus, OH (US); Seite 93, Nr. 140926q#

## Beschreibung

Die Erfindung betrifft die Verwendung bestimmter Carbonsäureester und mehrwertiger Alkohole als Extraktionsmittel zur Gewinnung reiner, 3-Hydroxybuttersäureeinheiten enthaltender Polyester oder Copolyester aus dem Zellmaterial eines, diese enthaltenden Mikroorganismus und ein Verfahren zur Extraktion von 3-Hydroxybuttersäureeinheiten enthaltender Polyester oder Copolyester aus dem Zellmaterial eines Mikroorganismus unter Verwendung dieser Extraktionsmittel.

Poly-D(-)-3-hydroxybuttersäure (Poly-HB) wird als Speicherstoff für Energie und Kohlenstoff von vielen Mikroorganismen zellintern aufgebaut und angehäuft und stellt einen Polyester mit thermoplastischen Eigenschaften dar, der biologisch abbaubar ist. Poly-HB kann beispielsweise nach der in EP-A-0 149 744 oder EP-A-0 144 017 beschriebenen Verfahrensweise in guten Ausbeuten problemlos hergestellt werden. Copolyester von Poly-HB, wie z.B. Copolyester, bestehend aus 3-Hydroxybuttersäure- und 3-Hydroxyvaleriansäureeinheiten oder auch anderen Säureeinheiten, sollen nach EP-A-0 052 459 bessere Verarbeitungseigenschaften bei der Verwendung als Thermoplasten aus reines Poly-HB aufweisen. Ein Verfahren zur Herstellung solcher Copolyester ist in der EP-A-0 069 497 geoffenbart.

Die Polyester liegen nach der biologischen Herstellung im Zellmaterial des Mikroorganismus vor und müssen dann aus dem Zellmaterial extrahiert werden. Dies bereitete bisher erhebliche Schwierigkeiten.

So ist beispielsweise ins US-A-3 036 959 oder in US-A-3 044 942 beschrieben, daß gute Ausbeuten bei der Extraktion der Polyester aus dem Zellmaterial des Mirkoorganismus nur dann erreicht werden können, wenn man dem eigentlichen Extraktionsschritt einen zusätzlichen Schritt zum Aufbrechen der Zellen vorschaltet, in dem die Zellen mit Aceton behandelt werden. Als Extraktionsmittel müssen Pyridin bzw. Methylenchlorid verwendet werden.

In der US-A-3 275 610 wird als Extraktionsmittel Chloroform beschrieben. Um gute Ausbeuten zu erreichen, müssen die Zellen jedoch sehr lange Zeit mit dem Extraktionsmittel behandelt werden. Durch die lange Behandlung tritt aber eine Depolymerisierung der Poly-HB auf, sodaß bei dieser Methode entweder eine schlechte Ausbeute oder ein Abbau des Molekulargewichtes von Poly-HB in Kauf genommen werden muß.

In der US-A-4 310 684 werden zur Extraktion andere halogenierte Kohlenwasserstoffe vorgeschlagen. Halogenierte Kohlenwasserstoffe sind aber insgesamt giftig und stellen eine Gefahr für jeden, der damit arbeiten muß, und zusätzlich eine Belastung für die Umwelt dar, wobei außerdem in Betracht zu ziehen ist, daß Restgehalte dieser Lösungsmittel in der isolierten Poly-HB unvermeidlich sind.

In US-A-4 101 533 wurden daher cyclische Kohlensäureester wie Ethylen- oder Propylencarbonat als Lösungsmittel für Polyhydroxybuttersäure vorgeschlagen. Diese Lösungsmittel sind jedoch im heißen Zustand, dem sie verwendet werden müssen, sehr aggressiv und greifen Hähne und Dichtungen von Apparaturen an. Für eine gute Ausbeute bei der Extraktion von Poly-HB ist eine längere Behandlung der Zellen mit Ethylen- oder Propylencarbonat notwendig, wobei jedoch ein besonders startker Abbau des Molekulargewichtes der Poly-HB oder deren Copolyester eintritt, was für die Anwendung der Poly-HB oder deren Copolyester als Thermoplast nachteilige Folgen hat.

Demgegenüber konnten nun Lösungsmittel zur einfachen und problemlosen Extraktion von Poly-HB und deren Copolyester gefunden werden, bei deren Verwendung als Extraktionsmittel die oben angeführten Nachteile vermieden werden, wobei die Polyster oder Copolyester in unerwartet hoher Reinheit von mindestens 98 % in sehr guten Ausbeuten anfallen.

Gegenstand der Erfindung ist demnach die Verwendung von Diolen oder acetalisierten Triolen, von Di- oder Tricarbonsäureestern, von Gemischen aus Dicarbonsäureestern oder von Butyrolacton als Extraktionsmittel zur Gewinnung reiner, 3-Hydroxybuttersäureeinheiten enthaltender Polyester oder Copolyester aus dem Zellmaterial eines, diese enthaltenden Mikroorganismus.

Die erfindungsgemäßen Diole können aliphatischge, geradkettige oder verzweigte Diole mit einer Kettenlänge von 2 bis 8 C-Atomen sein, wobei die aliphatische Kette durch ein oder 2 Stickstoffatome, die gegebenenfalls durch eine Methylgruppe substituiert sein können, durchbrochen sein kann. Bevorzugte Diole sind z. B. Propan-, Butan- und Hexandiole, Ethylhexandiole, N-Methyldiethanolamin, N,N-bis(2-Hydroxyethyl)-1,3-diamino-propan, wobei Propandiole besonders bevorzugt sind. Die erfindungsgemäßen acetalisierten Triole sind bevorzugt Glycerinformal oder 2,2-Dimethyl-4-hydroxymethyl-1,3-dioxolan, wobei Glycerinformal besonders bevorzugt ist.

Die Säurekomponente der erfindungsgemäßen Dicarbonsäureester kann aus geradkettigen oder verzweigten, gesättigten oder ungesättigten, aliphatischen oder aromatischen Dicarbonsäuren mit 2 bis 8 C-Atomen, wobei die aliphatische Kette durch ein- oder mehrere Hydroxy- oder durch eine Acetylgruppe substituiert sein kann oder aus Azodicarbonsäure bestehen. Als Alkoholkomponente der Dicarbonsäureester kommen geradkettige oder verzweigte Alkylalkohole in Betracht. Als Beispiele seien Methyl-, Ethyl-, Propyl-, Butyl-, Pentyl-und Hexylalkohole und ihre Isomeren genannt. Die erfindungsgemäßen Tricarbonsäureester sind Zitronensäureester, wobei als Alkoholkomponente z. B. die oben angeführten Alkohole in Frage kommen, oder Triacetin.

Die Di- oder Tricarbonsäureester können symmetrische oder unsymmetrische, bevorzugt symmetrische Ester sein. Bevorzugte Dicarbonsäureester sind beispielsweise Oxalsäurediethylester, Malonsäuredimethyl- und -diethylester, Bernsteinsäuredimethyl- und -diethylester, Glutarsäuredimethylester, Adipinsäuredimethylester, Ethylmalonsäurediethylester, Maleinsäuredimethylester, Fumarsäuredibutylester, Phthalsäurediethylester, Weinsäurediethyl- und -dibutylester, Acetylbernsteinsäuredimethylester und Azodicarbonsäurediisopropylester, wobei Bernsteinsäuredimethyl- und -diethylester, besonders bevorzugt sind.

Die erfindungsgemäßen Extraktionsmittel können auch Gemische der beschriebenen Dicarbonsäureester sein. Ein besonders bevorzugtes Gemisch setzt sich aus Bernsteinsäure-, Glutarsäure- und Adipinsäuredimethylester zusammen, bevorzugt im Verhältnis 1 : 4 : 1. Unter Butyrolacton ist gamma-Butyrolacton zu verstehen.

Zur Gewinnung der reinen Polyester oder Copolyester wird das Zellmaterial des Mikroorganismus, das die 3-Hydroxybuttersäureeinheiten enthaltenden Polyester oder Copolyester enthält, durch übliche Methoden, bevorzugt durch Abzentrifugieren der Fermenterlösung, aus dem Fermenter bzw. aus der Fermenterlösung isoliert. Das abgetrennte Zellmaterial kann wie üblich getrocknet werden, oder man setzt das Zellmaterial wasserfeucht in den Extraktionsschritt ein. Bevorzugt wird das Zellmaterial wasserfeucht eingesetzt, wobei der Wassergehalt des Zellmaterials in allgemeinen 40 bis 80 Gew.% beträgt. Das aus dem Fermenter isolierte Zellmaterial wird in einem der erfindungsgemäßen Extraktionsmittel aufgerührt und auf Temperaturen von etwa 100 bis 150 °C erhitzt und 5 bis 20 Minuten bei dieser Temperatur gerührt. Anschließend wird das ungelöste Zellmaterial vom heißen Extraktionsmittel, das die Polyester gelöst enthält, abgetrennt. Die Abtrennung kann durch übliche Methoden erfolgen, wobei die Verwendung beheizter Saugnutschen vorteilhaft ist, da die Abtrennung auf diese Weise überraschend problemlos und einfach gelingt. Die abgetrennte Lösung, die die Polyester enthält, wird danach abgekühlt, wobei die Polyester ausgelieren oder die Polyester werden durch Zugabe von Fällungsmitteln wie etwa Wasser, Methanol, Ethanol, Aceton oder Mischungen davon kristallin ausgefällt. Die Isolierung der Polyester erfolgt etwa durch Abfiltrieren, Absaugen, Abzentrifugieren oder Abpressen der Flüssigkeit aus Gelen. Der isolierte Niederschlag wird mit Wasser, Methanol, Ethanol, Aceton oder Mischungen davon nachgewaschen, wobei ausgefallene Gele kristallisieren, und getrocknet. Die Trocknung der Polyester erfolgt auf übliche Weise, beispielsweise im Trockenschrank.

In einer bevorzugten Ausführungsform wird das Zellmaterial des Mikroorganismus von der Fermenterlösung abzentrifugiert, in Propandiol, Glycerinformal, Bernsteinsäuredimethyl- oder Bernsteinsäurediethylester, einem Estergemisch bestehend aus Bernsteinsäure-, Glutarsäure- und Adipinsäuredimethylester oder in Butyrolacton auf 110 bis 140 °C erhitzt und 15 Minuten bei dieser Temperatur gerührt. Das ungelöste Zellmaterial wird mittels einer beheizten Saugnutsche abgetrennt und die heiße Lösung abgekühlt, wobei die Polyester ausgelieren. Die Lösung kann aber auch mit einem Fällungsmittel wie Wasser, Ethanol, Methanol, Aceton oder Mischungen davon versetzt werden, wobei die Polyester kristallin ausfallen. Die Gele werden durch Absaugen isoliert, wobei die Flüssigkeit gegebenenfalls noch zusätzlich abgepreßt werden kann und durch Verrühren des Rückstandes mit einem Fällungsmittel wie Wasser, Ethanol, Methanol, Aceton oder Mischungen davon kristallisiert; der kristalline Niederschlag wird abgesaugt und getrocknet. Da die Extraktionsmittel gegenüber den Fällungsmitteln durchwegs hohe Siedepunkte aufweisen, können sie von den Fällungsmitteln durch beispielsweise Destillation rückgewonnen werden und sowohl die Fällungs- als auch die Extraktionsmittel können immer wieder von neuem in die Extraktion eingesetzt werden.

Die Verwendung der erfindungsgemäßen Extraktionsmittel liefert Polyester in unerwartet hoher Reinheit von mindestens 98 % in sehr guten Ausbeuten, wobei nur eine außergewöhnlich geringe Depolymerisation der Polyester auftritt, und wobei die Extraktion einfach und problemlos auszuführen ist und stellt somit eine Bereicherung der Technik dar.

### Beispiel 1

100 g eines wasserfeuchten Zellmaterials aus dem Fermenter, gewonnen durch Abzentrifugieren der Fermenterlösung, mit einem Wassergehalt von 60 Gew.% und einem Poly-HB-Gehalt von 78% bezogen auf das Zelltrockengewicht, wurden mit 360 g 1,2-Propandiol 10 Minuten bei 140 °C gerührt. Nach Abtrennung des ungelösten Zellmaterials mittels einer beheizten Absaugnutsche wurde die Lösung abgekühlt, wobei Poly-HB ausgelierte. Das ausgefallene Gel wurde abgesaugt, mit Wasser durchgerührt und nachgewaschen, wobei das Gel kristallisierte, der kristalline Niederschlag abgesaugt und getrocknet. Dabei wurden 24,6 g Poly-HB, das entspricht 79 % der Theorie, mit einer Reinheit von 99,1 % und einem Molekulargewicht von 585.000 erhalten, wobei das Molekulargewicht der Poly-HB in der Zellmasse 650.000 betrug.

### Beispiel 2

25 g eines wasserfeuchten Zellmaterials aus dem Fermenter, gewonnen durch Abzentrifugieren der Fermenterlösung, mit einem Wassergehalt von 60 Gew.% und einem Poly-HB-Gehalt von 65 % bezogen auf das Zelltrockengewicht, wurden mit 390 g Glycerinformal bei 120 °C 15 Minuten gerührt. Nach Abtrennung des ungelösten Zellmaterials mittels einer beheizten Absaugnutsche wurde die Lösung abgekühlt, wobei Poly-HB ausgelierte. Das ausgefallene Gel wurde abgesaugt und mit Wasser und Aceton nachgewaschen, wobei das Gel kristallisierte, der kristalline Niederschlag abgesaugt und getrocknet. Dabei wurden 5,5 g Poly-HB, das entspricht 85 % der Theorie, mit einer Reinheit von 99,7 % und einem Molekulargewicht von 700 000 erhalten, wobei das Molekulargewicht der Poly-HB in der Zellmasse 780.000 betrug.

### Beispiel 3

25 g eines wasserfeuchten Zellmaterials aus dem Fermenter, gewonnen durch Abzentrifugieren der Fermenterlösung, mit einem Wassergehalt von 60 Gew.% und einem Poly-HB-Gehalt von 62% bezogen auf das Zelltrockengewicht, wurden mit 390 g Bernsteinsäurediethylester 15 Minuten bei 110 °C gerührt. Nach Abtrennung des ungelösten Zellmaterials mittels einer beheizten Absaugnutsche, wurde die Lösung abgekühlt, wobei Poly-HB ausgelierte. Das ausgefallene Gel wurde abgesaugt, mit Wasser und Ethanol nachgewaschen, wobei das Gel kristallisierte, der kristalline Niederschlag abgesaugt und getrocknet. Dabei wurden 5,6 g Poly-HB, das entspricht 90 % der Theorie, mit einer Reinheit von 100 % und einem Molekulargewicht von 400.000 erhalten, wobei das Molekulargewicht der Poly-HB in der Zellmasse 470.000 betrug.

### Beispiel 4

25 g eines wasserfeuchten Zellmaterials aus dem Fermenter, gewonnen durch Abzentrifugierten der Fermenterlösung, mit einem Wassergehalt von 60 Gew.% und einem Poly-HB-Gehalt von 62 % bezogen auf das Zelltrockengewicht, wurden mit 390 g Bernsteinsäuredimethylester 15 Minuten bei 110 °C gerührt. Nach Abtrennung des ungelösten Zellmaterials mittels einer beheizten Absaugnutsche, wurde die Lösung abgekühlt und mit Methanol versetzt, wobei Poly-HB ausgefällt wurde. Der ausgefallene Niederschlag wurde abgesaugt, mit Wasser und Aceton nachgewaschen und getrocknet. Dabei wurden 5,3 g Poly-HB, das entspricht 86 % der Theorie, mit einer Reinheit von 99,9 % und einem Molekulargewicht von 420.000 erhalten, wobei das Molekulargewicht der Poly-HB in der Zellmasse 470.000 betrug.

### Beispiel 5

25 g eines wasserfeuchten Zellmaterials aus dem Fermenter, gewonnen durch Abzentrifugieren der Fermenterlösung, mit einem Wassergehalt von 60 % und einem Poly-HB-Gehalt von 62 % bezogen auf das Zelltrockengewicht, wurden mit 390 g einer Mischung bestehend aus Bernsteinsäuredimethylester : Glutarsäuredimethylester : Adipinsäuredimethylester im Verhältnis 1 : 4 : 1 15 Minuten bei 120 °C gerührt. Nach Abtrennung des ungelösten Zellmaterial mittels einer beheizten Absaugnutsche wurde die Lösung abgekühlt und mit Ethanol versetzt, wobei Poly-HB ausfiel. Der Niederschlag wurde abgesaugt, mit Ethanol nachgewaschen und getrocknet. Dabei wurden 5,5 g Poly-HB, das entspricht 89 % der Theorie mit einer Reinheit von 98,9 % und einem Molekulargewicht von 725.000 erhalten, wobei das Molekulargewicht der Poly-HB in der Zellmasse 780.000 betrug.

### Beispiel 6

125 g eines wasserfeuchten Zellmaterials aus dem Fermenter, gewonnen durch Abzentrifugieren der Fermenterlösung, mit einem Wassergehalt von 60 Gew.% und einem Poly-HB-Gehalt von 62 % bezogen auf das Zelltrockengewicht, wurden mit 450 g Butyrolacton bei 110 °C 15 Minuten gerührt. Nach Abtrennung des ungelösten Zellmaterials mittels einer beheizten Absaugnutsche, wurde die Lösung abgekühlt, wobei Poly-HB ausgelierte. Das ausgefallene Gel wurde abgesaugt und mit Wasser und Aceton nachgewaschen, wobei das Gel kristallisierte, der kristalline Niederschlag abgesaugt und getrocknet. Dabei wurden 28 g Poly-HB, das entspricht 90 % der Theorie, mit einer Reinheit von 99,5 % und einem Molekulargewicht von 735.000 erhalten, wobei das Molekulargewicht der Poly-HB in der Zellmasse 780.000 betrug.

### Beispiel 7

50 g eines wasserfeuchten Zellmaterials aus dem Fermenter, gewonnen durch Abzentrifugieren der Fermenterlösung mit einem Wassergehalt von 60 Gew.% und einem Gehalt eines Copolyesters von 77,3 % bezogen auf das Zelltrockengewicht, wobei der Copolyester aus 98,1 % D(-)-3-Hydroxybuttersäure- und aus 1,9 % 3-Hydroxyvaleriansäureeinheiten zusammengesetzt war, wurden mit 400 g Bernsteinsäuredimethylester bei 110 °C 15 Minuten gerührt. Nach Abtrennung des ungelösten Zellmaterials mittels einer beheizten Absaugnutsche, wurde die Lösung abgekühlt und der Copolyester durch Zugabe von Methanol ausgefällt. Der ausgefallene Niederschlag wurde abgesaugt und mit Wasser und Aceton nachgewaschen. Dabei wurden 12,3 g Copolyester, das entspricht 80 % der Theorie, der aus 98,1 % D(-)-3-Hydroxybuttersäure- und aus 1,9 % 3-Hydroxyvaleriansäureeinheiten zusammengesetzt war, mit eine Reinheit von 100 % und einem Molekulargewicht von 770.000 erhalten, wobei das Molekulargewicht des Copolyesters in der Zellmasse 800.000 betrug.

### Beispiel 8

50 g eines wasserfeuchten Zellmaterials aus dem Fermenter, gewonnen durch Abzentrifugieren der Fermenterlösung, mit einem Wassergehalt von 60 Gew.% und einem Gehalt eines Copolyesters von 77,3 % bezogen auf das Zelltrockengewicht, wobei der Copolyester aus 98,1 % D(-)-3-Hydroxybuttersäure- und aus 1,9 % 3-Hydroxyvaleriansäureeinheiten zusammengesetzt war, wurden mit 400 g einer Mischung bestehend aus Bernsteinsäuredimethylester : Glutarsäuredimethylester : Adipinsäuredimethylester im Verhältnis 1 : 4 : 1 bei 110 °C 15 Minuten gerührt. Nach Abtrennung des ungelösten Zellmaterials mittels einer beheizten Absaugnutsche, wurde die Lösung abgekühlt und der Copolyester durch Zugabe von Methanol ausgefällt. Der ausgefallene Niederschlag wurde abgesaugt, mit Wasser und Aceton nachgewaschen und getrocknet. Dabei wurden 13,9 g Copolyester, das entspricht 90 % Theorie, der aus 98,1 % D(-)3-Hydroxybuttersäure- und aus 1,9 % 3-Hydroxyvaleriansäureeinheiten zusammengesetzt war, mit eine Reinheit von 100 % und einem Molekulargewicht von 785.000 erhalten, wobei das Molekulargewicht des Copolyesters in der Zellmasse 800.000 betrug.

In den Beispielen erfolgte die Polyhydroxybuttersäure-Bestimmung nach der Methode von Braunegg et al., Eur. J. Appl. Microbiol. Biotechnol. 6, 29 bis 37, (1978).
Die Molekulargewichtsbestimmung wurde mittels Gelchromatographie (PL Microgel M, 60 cm Säule, 1 g/l in Chloroform, 1 ml/min, Polystyrolstandards, Dichtedetektion) ausgeführt. Der Wassergehalt wurde durch Trockenverlust bestimmt.

## Patentansprüche

1. Verwendung von Polen oder acetalisierten Triolen, von Di- oder Tricarbonsäureestern, von Gemischen von Dicarbonsäureestern oder von Butyrolacton als Extraktionsmittel zur Gewinnung reiner, 3-Hydroxybuttersäureeinheiten enthaltender Polyester oder Copolyester aus dem Zellmaterial eines, diese enthaltenden Mirkoorganismus.

2. Verwendung nach Anspruch 1, dadurch gekennzeichnet, daß als Extraktionsmittel Propandiol eingesetzt wird.

3. Verwendung nach Anspruch 1, dadurch gekennzeichnet, daß als Extraktionsmittel Glycerinformal eingesetzt wird.

4. Verwendung nach Anspruch 1, dadurch gekennzeichnet, daß als Extraktionsmittel Bernsteinsäuredimethyl- oder Bernsteinsäurediethylester eingesetzt wird.

5. Verwendung nach Anspruch 1, dadurch gekennzeichnet, daß als Extraktionsmittel ein Gemisch aus Bernsteinsäure-, Glutarsäure- und Adipinsäuredimethylester eingesetzt wird.

6. Verwendung nach Anspruch 1, dadurch gekennzeichnet, daß als Extraktionsmittel Butyrolacton eingesetzt wird.

7. Verfahren zur Gewinnung reiner, 3-Hydroxybuttersäureeinheiten enthaltender Polyester oder Copolyester aus dem Zellmaterial eines, diese enthaltenden Mikroorganismus, dadurch gekennzeichnet, daß das Zellmaterial mit Polen, acetalisierten Triolen, Di- oder Tricarbonsäureestern, Gemischen von Dicarbonsäureestern oder Butyrolacton extrahiert wird.

## Claims

1. Use of diols or acetalized triols, di- or tricarboxylic acid esters, mixtures of dicarboxylic acid esters or butyrolactone as extracting agents for obtaining pure polyesters or copolyesters containing 3-hydroxybutyric acid units form the cell material of a microorganism containing these.

2. Use according to Claim 1, characterized in that propanediol is employed as the extracting agent.

3. Use according to Claim 1, characterized in that glycerol formal is employed as the extracting agent.

4. Use according to Claim 1, characterized in that dimethyl succinate or diethyl succinate is employed as the extracting agent.

5. Use according to Claim 1, characterized in that a mixture of dimethyl succinate, glutarate and adipate is employed as the extracting agent.

6. Use according to Claim 1, characterized in that butyrolactone is employed as the extracting agent.

7. Process for obtaining pure polyesters or copolyesters containing 3-hydroxybutyric acid units from the cell material of a microorganism containing these, characterized in that the cell material is extracted with diols, acetalised triols, di- or tricarboxylic acid esters, mixtures of dicarboxylic acid esters or butyrolacetone.

## Revendications

1. Utilisation de diols ou de triols acétalisés, d'esters d'acides di- ou tricarboxyliques, de mélanges d'esters d'acides dicarboxyliques ou de butyrolactone, comme des agents d'extraction pour l'obtention de polyesters ou de copolyesters purs contenant des unités d'acide 3-hydroxybutyrique, à partir du matériau cellulaire d'un micro-organisme contenant celles-ci.

2. Utilisation selon la revendication 1, caractérisée en ce qu'on utilise le propanediol comme agent d'extraction.

3. Utilisation selon la revendication 1, caractérisée en ce qu'on utilise le glycérine-formal comme agent d'extraction.

4. Utilisation selon la revendication 1, caractérisée en ce qu'on utilise l'ester diméthylique de l'acide succinique ou l'ester diéthylique de l'acide succinique comme agent d'extraction.

5. Utilisation selon la revendication 1, caractérisée en ce qu'on utilise un mélange des esters diméthyliques de l'acide succinique, de l'acide glutarique et de l'acide adipique comme agent d'extraction.

6. Utilisation selon la revendication 1, caractérisée en ce qu'on utilise la butyrolactone comme agent d'extraction.

7. Procédé pour l'obtention de polyesters ou de copolyesters purs contenant des unités d'acide 3-hydroxybutyrique à partir du matériau cellulaire d'un micro-organisme contenant celles-ci, caractérisé en ce qu'on extrait le matériau cellulaire avec des diols, des triols acétalisés, des esters d'acides di- ou tricarboxyliques, des mélanges d'esters d'acides dicarboxyliques, ou la butyrolactone.
